# EUROPEAN PATENT SPECIFICATION

(11) **EP 1 663 172 B1**
(45) Date of publication and mention of the grant of the patent: **21.05.2008**
(21) Application number: 04766400.8
(22) Date of filing: 03.08.2004
(51) Int. Cl.: A61K 9/48

(54) **POLYSACCHARIDE DOUBLE-LAYER MICROCAPSULES AS CARRIERS FOR BIOLOGICALLY ACTIVE SUBSTANCE ORAL ADMINISTRATION**
POLYSACCHARID-DOPPELSCHICHT-MIKROKAPSELN ALS TRÄGER FÜR BIOLOGISCHE WIRKSUBSTANZ ZUR ORALEN VERABREICHUNG
MICROCAPSULES A DOUBLE COUCHE DE POLYSACCHARIDES UTILISEES EN TANT QU'EXCIPIENTS POUR L'ADMINISTRATION PAR VOIE ORALE DE SUBSTANCES BIOLOGIQUEMENT ACTIVES

(30) Priority: 06.08.2003 IT MI20031617
(43) Date of publication of application: 07.06.2006
(73) Proprietor: Fondazione Carlo e Dirce Callerio Onlus, 34127 Trieste (IT)
(72) Inventor: SAVA, Gianni, I-34143 Trieste (IT); ZORZIN, Laura, I-33050 Fiumicello (IT); VOJNOVIC, Dario, I-34128 Trieste (IT)
(74) Representative: Gervasi, Gemma
(86) International application number: PCT/EP2004/051693
(87) International publication number: WO 2005/013941

(56) References cited:
- GB-A- 1 236 885
- US-A- 5 116 747
- US-B1- 6 234 464

## Description

### Field of the invention

The invention relates to double-layer microcapsules of chitosan and alginate gelifed and stabilized with a divalent ion, incorporating at least one biologically active substance, which can be employed usefully for oral vaccinogenic or therapeutic purposes in the human and veterinary field.

### State of the art

In order for therapeutic agents, such as proteins or peptides, which are commonly only administrable parenterally, to be efficiently carried orally, for some time research has been oriented towards the preparation of microcapsules capable of incorporating and releasing said agents in a controlled manner.

Among the polymers employable to prepare microcapsule, natural polysaccharides, such as starch, κ-carrageenan, alginate, agar, agarose, dextran and chitosan are particularly interesting for their chemico-physical properties and for their high level of biocompatibility and biodegradability. Polysaccharides are in fact non-toxic polymers which can form gel-like structures, in which it is possible to encapsulate therapeutic, or in any case biologically active agents, even with high molecular weight.

Moreover, it is known that polysaccharides have bioadhesive properties, which is a particularly important characteristic for efficient therapeutic absorption through the gastric or intestinal mucosa of the encapsulated active ingredients.

Both chitosan and alginate alone, combined with each other or with other polymers have been studied for encapsulation of protein, as in an aqueous environment they can gel and form microcapsules. For alginate this process is enhanced by the presence of divalent ions, calcium ions in particular.

US 5116747 discloses the preparation of microcapsules for biological material comprising chitosan and alginate.

Polk. A. et al. (1994 J. Pharm. Sci., 83:178-185) describes the preparation of microcapsules containing albumin with different concentrations of alginic acid (from 1.5 to 2.5% w/v), chitosan (from 0.1 to 0.4% w/v) and calcium chloride (1.5% w/w) obtaining microcapsules with a diameter of approximately 250 µm in dry state and studies the chemico-physical properties and release of the albumin.

More recently Vandenberg G.W. et al. (2001 J. Control. Release, 77: 297-307) studied the influence of various factors on the release of albumin from alginate and chitosan coacervates, gelified with calcium chloride, in basal conditions, with an acid (1.5) and neutral (7.5) pH, in order to identify the best conditions for developing an encapsulation carrier system for proteins. The standard protocol for albumin encapsulation was: an aqueous solution of alginate (2% w/v), to which 25% (protein mass : alginate mass) of albumin was added, was extruded in a solution of chitosan (2% w/v) in acetic acid, the solution of which was taken to pH 5.5 and to which calcium chloride (1.5% w/v) was added. The various factors studied with respect to this encapsulation protocol were: i) various loading percentages of the protein (25, 50, 75, 100 % protein mass : alginate mass w/w); ii) various pH (3, 4, 5, 6) of the medium during encapsulation or iii) different concentrations of the three components. In particular, in this last condition protein retention was tested in concentrations ranging from 1.0 to 3.0 (1.0, 1.5, 2.0, 2.5, 3.0) % w/v of alginate, from 0 to 0.75% (0, 0.125, 0.25, 0.375, 0.5, 0.75) w/v of chitosan and from 0.05 to 5 (0.05, 0.1, 0.5, 1, 1.5, 5) % w/v of CaCl₂. The experimental data indicate that the optimal concentration of alginate for protein encapsulation is 2%, while its retention at acid pH reaches the maximum at an alginate concentration of 2.5%. For chitosan the optimal concentrations were 0.125% for encapsulation and 0.25% for protein retention at acid pH, while variation of the calcium concentrations do not seem significant to influence the two parameters considered, as the best concentration for calcium for both parameters is found to be 0.5%. The most suitable protein loading is at 25%. It is nonetheless important to observe that in an acid environment in conditions simulating gastric conditions with the microcapsules described there is always significant protein release, while in neutral conditions this does not seem to be influenced.

To develop efficient oral carrier systems for biologically active substances, particular attention has to be paid to release at intestinal level. Intestinal absorption can in fact be preferred for substances easily degradable in an acid environment or for substances suitable for an immune response production, release which, associated with the ability to adhere to the mucosa of the microcapsules employed to carry said substances, can determine a significant increase in the response. In this case it is in fact known that the intestinal mucosa has a large number of lymphoid cells able to produce an effective immune response to external infective stimuli (Van der Lubben I.M. et al., 2001 Adv. Drug. Del. Rev., 52: 139-144*;* Schep L.J. et al., 1999 J.Contr. Release, 59: 1-14).

Moreover, this process does not seem to be a peculiarity confined to mammals alone. In fact, for example, interesting data on this subject is reported in literature in fish, although the exact nature of the systemic immune response following oral administration in fish is not yet fully understood. Oral release systems, suitable to protect the antigen in the stomach of fish and to allow uptake at intestinal level, where it can be processed by the competent cells, are capable of evoking a protective immune response (*Schep L.J., et al. 1999 ref. cit*.). With reference to the local immune response it has been observed that the second segment of the terminal intestine of fish is able to absorbe soluble or particulate antigens (*Schep L.J., et al 1999 ref. cit*.)*.* Furthermore, specific antibodies have been found in various orally vaccinated fish species such as: trout, bass, catfish and flounders (Ainsworth AJ et al. 1995 J. Fish Disease, 18 (5): 397- 409). In order to obtain efficient systems for intestinal absorption following oral administration it is therefore essential that: i) the microcapsules encapsulating the biologically activated substances are not significantly degraded in an acid environment at gastric level protecting said substances from degradation and there is no significant release in an acid environment, thus allowing said substances to reach the intestine; ii) the outer surface of the microcapsules has good bioadhesive capacities so that they can adhere to the intestinal mucosa and release the encapsulated biologically active substances in a controlled way. The microcapsules must also encapsulate one or more substances so as to induce an adequate biological response and their dimensions must be extremely small (<10µm) so as to allow uptake by the Peyer's patches. Uptake by the Peyer's patches is particularly important in the case in which the biologically active substances are antigens administered for the purpose of inducing an immune response.

It is also important for the oral carrier system as a whole to be easily administered and produced at industrial level and for it to have moderate costs.

Although studies on release from alginate and chitosan coacervates by Vandenberg G.W. et al. (*ref. cit.*) are of definite interest, the microcapsules described do not seem to have the characteristics required to develop an oral carrier system with the aforesaid characteristics, in relation to the fact that release of the encapsulated protein - in this particular case albumin - essentially takes place at acid pH.

### Summary of the invention

The purpose is to develop a new system for oral administration of biologically active substances, suitable to determine absorption essentially at intestinal level, in natural polysaccharide microcapsules which have the characteristics of: i) encapsulating at least one biologically active substance so that it is protected from degradation in an acid environment at gastric level; ii) allowing release of said substance at intestinal level; iii) having good bioadhesive capacities so that it adheres to the intestinal mucosa and releases said substance in an controlled way; iv) having a very small dimension to allow uptake by the Peyer's patches, particularly in the case of antigens administered for vaccinogenic purposes and of enzymatically degradable molecules.

Moreover, the system must be able to encapsulate one or more substances so that an adequate biological response is obtained from their release.

For this purpose it has surprisingly been found that suitable and unexpected concentration ratios between alginate/chitosan polysaccharides and even more so between alginate/chitosan polysaccharides and one divalent ion in the microcapsule preparation have particular relevance for the stability thereof and for the release of one or more biologically active substances contained therein at intestinal level, and for the dimensions of said microcapsules. Moreover, the addition of a third polymer, such as hydroxypropylmethylcellulose (HPMC), is preferential for the purposes of the present invention.

The object of the present invention are therefore microcapsules with a double-layer of polysaccharides constituted by an outer layer of chitosan and an inner layer of alginate, obtained:
- from solutions of alginate with initial concentrations ranging from 2 to 4% w/v;
- from solutions of chitosan with initial concentrations ranging from 0.1 to 0.5 % w/v;
- from solutions of divalent ions with concentrations of 0.5% w/v, when the divalent ion functions as a gelification agent of the alginate to form single-layer capsules of alginate encapsulating at least one biologically active substance and ranging from 10 to 15% w/v when the divalent ion has a stabilizing function of the double-layer capsules.

Further objects of the present invention include: i) the process for preparing said microcapsules, ii) the compositions for their administration and iii) their use as carriers for oral administration of biologically active substances for the prophylaxis and therapy of infectious or non-infectious diseases in the human and veterinary field.

### Brief description of the figure

Figure 1: A) Photo of double-layer microcapsules; B) enlarged detail of these microcapsules.
Figure 2: Effect of the variation in the concentration of chitosan, alginate, calcium chloride and HPMC in the microencapsulation process expressed as a percentage w/v: A) effect on the loading fraction, B) effect on total release of the lysozyme from the microcapsule.

### Detailed description of the invention

The characteristics and advantages of the microcapsules shall be better understood by reading the following description, wherein said microcapsules with double-layer of chitosan/alginate shall be described as possible embodiments. The examples described shall therefore be provided purely for illustrative purposes as non-limiting examples of the invention.

In the development of an efficient oral carrier system for biologically active substances, particular attention was dedicated to the choice of polymers to prepare the microcapsules with regard to their stability in an acid environment and their capacity to release the biologically active substances at intestinal level.

With reference to this aspect, natural polysaccharides were preferred among the polymers for their high biocompatibility and non-toxicity, in addition to their chemico-physical properties and their various industrial applications. Chitosan and alginate, polysaccharides being able to form gels in an aqueous environment and already widely used in the food and pharmaceutical industry, were chosen for the purpose. Moreover, chitosan is suitable for its mucoadhesive properties and because it can be used to obtain, with appropriate techniques, such as the spray dry technique, microcapsules with dimensions of <5µm (optimal diameter for uptake of the microcapsules at the level of the intestinal mucosa). Alginates are currently used in various pharmacology and biotechnology fields with applications that range from controlled release of drugs to encapsulation of enzymes and/or cells. It is also known that alginate, in the presence of divalent ions, such as magnesium, zinc and calcium, can form gels which tend to precipitate in the presence of an excess of these ions. For the purposes of the present invention the calcium ion is preferred.

The microcapsules of the present invention are constituted by a double polymeric layer, with a polycationic outer coating of chitosan and a polyanionic inner layer of alginate in which, if added, HPMC is interdispersed, the inner layer of which encapsulates at least one biologically active substance.

When the object is the development of a vaccinogenic system, the biologically active substance can be a biologically active substance able to inducing an immune response, an antigen or an antigen associated with an adjuvant.

The antigens can be chosen for example from: preparations with microorganisms killed by means of chemical or physical agents, preparations with avirulent mutants selected by means of differential culture, preparations with detoxified toxins, preparations with bacterial fractions, synthetic vaccines constituted by specific epitopes and anti-viral vaccines. Moreover, in this case, according to the type of response which is to be evoked, a specific adjuvant capable of increasing biological response can be associated with them.

Lysozyme is a particularly interesting biologically active substance for the objects of the present invention. In fact, lysozyme is a 14,000-Dalton highly basic globular protein, the antiviral, antibacterial and immunomodulant properties of which have been known for some time (Lysozyme: Model Enzymes in Biochemistry and Biology, edited by P.Jollès.- Birkhäuser 1996). Moreover, a number of observations compete in defining the concrete possibility, at the level of the intestinal lymphatic system, of modulating an immune response against systemic targets using lysozyme.

Lysozyme from hen egg white (HEL) represents a well characterized antigen which, suitably processed and presented in association with the class II Major Histocompatibility Complex (MHC), can evoke activation of the response mediated by T helper lymphocytes *(*Oki A., Sercarz E. 1985 J. Exp. Med., 161: 897*;* Allen P.M. et al. 1984 Proceedings Nat. Acad. Sci. USA, 81: 2489). In particular, different regions have been identified on the HEL molecule able to modulate the responses of the T cells (*Allen P.M. et al. 1984 ref. cit*), highlighting that a class II MHC molecule and a simple globular protein can produce specific multiples or ligands for the T-cell receptor (Allen P.M. et al. 1985 J. Exp. Med., 162: 1264)*.*

Correspondingly, it is useful to underscore how HEL has been described to be able to produce a situation of systemic immunostimulation, both in experimental models *(*Reitano S. et al. 1982 Fed. Proceedings, 41: 608*)* and in clinical trials in humans *(*Cartei F. et al. 1991 Drug Invest., 4 : 51*).* This situation is associated, at least in mammals, with an interaction with the Peyer's patches *(*Namba Y. et al. 1981 Infec. & Imm., 31: 580*).*

Owing to its properties, lysozyme can be efficiently carried using the microcapsules of the invention alone, as biologically active substance, or also as adjuvant associated with any antigen. When, for example, the object is the development of a vaccinogenic system to obtain an adequate immune response, whatever the antigen chosen, the use of lysozyme is in fact preferential as adjuvant owing to its know properties cited above. However, other adjuvants capable of stimulating the T or B lymphocyte cells cannot be excluded, such as *Mycobacterium sp*., muramyl dipeptide, glucanes (yeast extracts), levamisole, BCG, *Corynebacterium parvum,* polynucleotides, lipopolysaccharides, or mitogens such as lectins o cytokines.

Instead, for therapeutic purposes the biologically active substances can be chemotherapeutics, cytokines or growth factors.

It is known that ionic interaction between chitosan and alginate makes it possible to obtain stabilization of the structure of microcapsules prepared with them, thus slowing down the release time, and that the presence of chitosan provides the system with a certain degree of mucoadhesion; nonetheless, the problem of the dimension of microcapsules encapsulating a biologically active substance, but above all their stability and release in an acid environment must be considered with particular attention in the light of the results obtained on release in an acid environment rather than in a neutral one by Vandenberg G.W. et al. (*ref. cit*.), cited above. In particular, to prepare microcapsules capable of encapsulating at least one biologically active substance, optionally associated with an adjuvant, in a quantity sufficient to induce the desired biological response, to protect them in an acid environment and to release them at intestinal level, the preferred characteristics of the polysaccharides are:
- chitosan: polymer in hydrosoluble form, with low molecular weight of approximately 150,000 Dalton, with a deacetylation degree from around 80 to 90% in concentrations ranging from 0.1 to 0.5% w/v;
- sodium alginate: polymer with molecular weight of approximately 200,000 Dalton, viscosity with a value of approximately 200 mPa, and a degree of purity from around 80 to 90%, in concentrations ranging from 2 to 4% w/v;
- calcium ion, in the preferred form of chloride, in concentrations ranging from 10 to 15% w/v.

Optionally, HPMC, preferably with a degree of 90 SH - 4000 SR and viscosity of 4000 at 0.4% w/v, can be added to the two aforesaid polysaccharides. HPMC has a positive contribution in controlling the diameter of the particles and the viscosity of the solution, with improvement of gelification and loading of the biologically active substances, such as an adjuvant and an antigen, and their retention as shall be evident from the results reported hereunder.

Moreover, for uptake at the level of the gastrointestinal mucosa, in particular at the level of the Peyer's patches, the diameter of the microcapsule must be below 10 µm.

The microcapsules according to the invention can be prepared with known methods such as by injection, with the spray dry technique or yet others. Preparation by emulsification is preferred for the objects of the invention.

The double-layer polysaccharide microcapsules were in any case prepared according to a procedure comprising the following characterizing steps:
a) formation of single-layer capsules encapsulating at least one biologically active substance starting from solutions of alginate in concentrations ranging from 2 to 4% w/v, in which said substance is dispersed, by gelification with a solution of a divalent ion at a concentration of 0.5% w/v;
b) formation of the second layer of chitosan and stabilization of the double-layer microcapsule obtained by adding a solution of chitosan in concentrations ranging from 0.1 to 0.5% w/v and containing a divalent ion in concentrations ranging from 10 to 15% w/v in the solution containing the alginate single-layer capsules encapsulating at least one biologically active substance obtained in a).

Optionally, in the preparation of single-layer microcapsules HPMC (0.4% w/v) can be added to the solution of alginate.

In the case of preparation of microcapsules according to the examples set forth below, the general method was by emulsification and was performed as follows:
*a) Preparation of the solutions:*
   *Solution A:* CaCl₂ (10, 12, 15 % w/v) was added to 30 ml of a solution of chitosan (0.1, 0.2, 0.5 % w/v), solubilized in water/acetic acid (0.5 % v/v); the solution thus obtained was taken to pH 5.5 with the addition of 1N NaOH.
   *Solution B:* 1 ml of Arlacel 1689 (surfactant) was added to 100 ml of sunflower oil and the solution was kept under stirring at 1000 rpm for 10 minutes.
   *Solution C:* 12 ml of HPMC (0.4 % w/v) (1 g in 100 ml of EtOH + 150 ml water) was added to 10 g of a solution of sodium alginate (2, 3, 4 % w/v); it was left under stirring for about 5 minutes and subsequently sonified for another 5 minutes.
   *Solution D:* 0.1 g of lysozyme and/or 0.1 g (or less) of antigen or other biologically active substances were suspended in 1 ml of water.
   *Solution E:* Solution of CaCl₂ 0.5 % + 1 % Tween 80 in 10 ml of water.
*b) Emulsification (in a shaft stirrer)*
   - Solution D was suspended in solution C (with or without HPMC).
   - It was kept under stirring for 10 minutes.
   - The suspension obtained was poured dropwise into solution B.
   - It was kept under stirring for another 10 minutes at 1000 rpm.
   - Solution E was added slowly.
   - It was kept under stirring for 5 minutes at 1000 rpm
   - Solution A was added.
   - It was kept under stirring for 1000 rpm.
   - Dehydration with 60 ml of isopropanol under stirring for 5 minutes.
*c) Isolation of microspheres*
   - The emulsion obtained was centrifuged for 10 minutes at 1100 xg.
   - It was vacuum filtered through cellulose nitrate filters with cut-off of 0.45 µm and the residue was washed with isopropanol.
*e) Drying*
   - Finally, the precipitate was oven dried at 37°C for 24 h.

In the end particles are obtained constituted by an outer coating of chitosan and an inner layer of alginate encapsulating the lysozyme and antigen, between which HPMC is dispersed. With this procedure it is possible to encapsulate in the microcapsule a quantity of substance equal to a maximum of 20% in weight with respect to the total weight of the microcapsule.

With the procedure described above microcapsules were prepared, suitably varying the chitosan/alginate/calcium chloride ± HPMC ratios according to the diagram in Table 1:

**Tab. 1**

| *variables* | *number of levels* | *levels* |
|---|---|---|
| HPMC | 2 | yes/no |
| chitosan | 3 | 0.1 |
| | | 0.2 |
| | | 0.5 |
| alginate | 3 | 2 |
| | | 3 |
| | | 4 |
| CaCl₂ | 3 | 10 |
| | | 15 |

With the variables indicated in the table the theoretical quantity of microencapsulated lysozyme is as follows (Table 2):

**Table 2**

| | % | % | % | *0.4%* | | % |
|---|---|---|---|---|---|---|
| *Exp* | *chitosan* | *alginate* | *CaCl2* | *HPMC* | *lysozyme* | *encapsulated* |
| | | | | | | *lysozyme** |
| 1 | 0.1 | 2 | 10 | + | 0.1g | 33 |
| 2 | 0.2 | 3 | 12 | + | " | 25 |
| 3 | 0.5 | 4 | 15 | + | " | 20 |
| 4 | 0.1 | 3 | 15 | - | " | 25 |
| 5 | 0.2 | 4 | 10 | - | " | 20 |
| 6 | 0.5 | 2 | 12 | - | " | 33 |
| 7 | 0.1 | 4 | 12 | + | " | 20 |
| 8 | 0.2 | 2 | 15 | + | " | 33 |
| 9 | 0.5 | 3 | 10 | + | " | 25 |

| | | | | | | |
|---|---|---|---|---|---|---|
| * % lysozyme = grams of lysozyme / (grams of lysozyme + grams of alginate). | | | | | | |

Set forth below are some examples of preparation of microcapsules prepared according to the general procedure described above, in which the components were combined together in different ratios according to Table 2 and according to preferred ratios in which chitosan is 0.1% w/v, alginate is 4% and the calcium ion employed with a stabilizing function is 15% w/v and encapsulating the lysozyme and an antigen or only the lysozyme.

### Example 1: preparation by emulsification of microcapsules of chitosan (0.1% w/v), alginate (4% w/v) containing lysozyme (0.1 g) and/or Vibrio anguillarum

### Materials

- Aqueous solution of sodium alginate 4% (Pronova): 0.4 g of sodium alginate was dissolved in 10 ml of water.
- Aqueous solution of HPMC (0.4% w/v) (Eigenmann - Veronelli ; Milan) (12 ml): 1 g of HPMC was dissolved in 100 ml of EtOH and 150 ml of water subsequently added.
- Solution of CaCl₂ 0.5% + 1% Tween 80 in water (Applied - Science Laboratories Inc) (10 ml).
- Solution of anhydrous CaCl₂ (15% w/v) (SIGMA): 4.5 g of CaCl₂ was solubilized in 30 ml of water and the solution filtered.
- Solution of chitosan CL 210 0.1% (Fluka): 30 mg of chitosan was added to 30 ml of a solution of anhydrous CaCl₂ 15% to obtain a final chitosan concentration of 0.1 % (w/v).
- Isopropanol (BDH, POOL, UK), (60 ml).
- Sunflower oil (ESPERIS S.P.A.), (100 ml).
- Esters of sorbitol and glycerol (surfactant Arlacel 1689, ESPERIS S.P.A.) (10 ml).
- *Vibrio anguillarum* 01 lyophilized, inactivated to heat (60°C for 15 minutes): 0.05 g or 0.1 g of lyophilized bacteria suspended in 1 ml of water.
- Lysozyme hydrochloride (SPA Milan) with purity of 100%: 0.1 g of protein solubilized in 1 ml of water.

### Procedure

100 ml of sunflower oil with 1% of Arlacel 1689 were mixed by mechanical stirrer at 1000 rpm for 10 minutes. Separately, 12 ml of solution of HPMC (0.4% w/v) was added to 10 g of sodium alginate (4% w/v); the solution formed was stirred and subsequently sonified for 5 minutes at a frequency of 97 KHz ± 6%. The solution constituted by alginate and HPMC was added dropwise to the mixture, constituted by sunflower oil and surfactant and stirred for 10 minutes. The protein and the bacteria were added to the solution of alginate and HPMC. Subsequently, 10 ml of a solution of CaCl₂ 0.5% + 1 % Tween 80 was added to the oily mixture and left under stirring for 5 minutes. 30 ml of the solution of chitosan 0.1% containing CaCl₂ was then added to form Ca - alginate, an insoluble compound that tended to precipitate and it was left to react under stirring for a further 10 minutes.

Subsequently, the mixture was dehydrated with 60 ml of isopropanol and left under stirring for a further 5 minutes. The mixture thus obtained was centrifuged for 10 minutes at 1100 xg to favour precipitation of the microspheres. The precipitate was vacuum filtered using cellulose nitrate filters with cut-off of 0.45 µm and the residue washed with isopropanol. The microcapsules were finally oven dried at 37°C for 24 h.

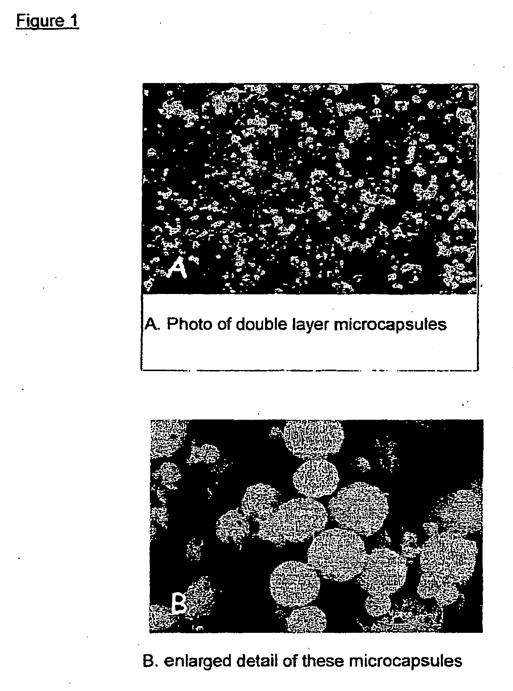

The microcapsules obtained are shown in Figure 1.

### Example 2: preparation by emulsification of microcapsules of chitosan (0.2% wlv), alginate (2% w/v) containing lysozyme with the addition of HPMC

### STEP 1

- Solution A: 4.5 g of CaCl₂ (15% w/v) was added to 30 ml of solution of chitosan (0.2% w/v) in water/adetic acid (0.5%); the solution obtained was taken to pH 5.5 with the addition of 1N NaOH.
- Solution B: 100 ml of sunflower oil and 1 ml of Arlacel (surfactant) were placed in a beaker and the solution kept under stirring at 1000 rpm for 10 minutes (shaft stirrer).
- Solution C: 12 ml of HPMC (0.4% w/v) (1 g in 100 ml of EtOH + 150 ml water) was added to 10 g of a solution of sodium alginate (2% w/v); this was left under stirring for about 5 minutes and subsequently sonified for another 5 minutes.
- Solution D: 0.1 g of lysozyme was resuspended in 1 ml of water.
- Solution E: 10 ml of CaCl₂ 0.5 % and 1 % Tween 80.

### STEP 2

Once the 4 solutions were prepared solution D (lysozyme) was added to the solution of alginate C and HPMC and left under stirring until obtaining a homogeneous suspension. Subsequently, the solution obtained was poured dropwise into solution B under stirring and left under stirring for 10 minutes. Solution E was then poured slowly into the beaker and left under stirring for 5 minutes. Subsequently solution A (chitosan and calcium chloride) was added and left under stirring for 10 minutes. Finally, 60 ml of isopropanol was added and it was left under stirring for 5 minutes. The emulsion obtained was centrifuged for 10 minutes at 1100 xg. It was vacuum filtered through cellulose nitrate filters with cut-off of 0.45 µm and the residue was washed with isopropanol. The filtered microcapsules were oven dried for 24 h at 37°C.

### Example 3: preparation by emulsification of microcapsules of chitosan (0.2% w/v), alginate (4% w/v) containing lysozyme without HPMC

### STEP 1

- Solution A: 3 g of CaCl₂ (10% w/v) were added to 30 ml of a solution of chitosan (0.2% w/v) solubilized in water/acetic acid (0.5%); the solution obtained was taken to pH 5.5 with the addition of NaOH.
- Solution B: 100 ml of sunflower oil and 1 ml of Arlacel (surfactant) were placed in a beaker and the solution kept under stirring at 1000 rpm for 10 minutes (shaft stirrer).
- Solution C: 10 gr of a solution of sodium alginate (4% w/v).
- Solution D: 0.1 g of lysozyme was resuspended with 1 ml of water.
- Solution E: 10 ml of CaCl₂ 0.5 % and Tween 80 (1%).

### STEP 2

Once the 4 solutions were prepared solution D (lysozyme) was added to the solution of alginate C and left under stirring until obtaining a homogeneous suspension. Subsequently, the solution obtained was poured dropwise into solution B under stirring and left under stirring for 10 minutes. Solution E was then poured slowly into the beaker and left under stirring for 5 minutes. Subsequently solution A (chitosan and calcium chloride) was added and left under stirring for 10 minutes. Finally, 60 ml of isopropanol was added and left under stirring for 5 minutes. The emulsion obtained was centrifuged for 10 minutes at 1100 xg. It was vacuum filtered through cellulose nitrate filters with cut-off of 0.45 µm and the residue was washed with isopropanol. The filtered microcapsules were oven dried for 24 h at 37°C.

For comparative purposes microcapsules were also prepared with only chitosan or alginate containing lysozyme or vibrio according to examples 4 and 5 set forth below.

### Example 4: Chitosan microspheres containing lysozyme or vibrio

1 g of HPMC was solubilized in 250 ml of ethanol (50% w/v) and 0.1 g of lysozyme or *Vibrio anguillarum,* suspended in 1 ml of water, were added. The solution obtained was mixed with a solution containing 1 g of chitosan in 100 ml of distilled water with glacial acetic acid (0.5% v/v); this was sonified for 15 minutes and then spray-dried. The process parameters were: flow velocity 0.25 l/h, inlet temperature 90°C, outlet temperature 60°C, air flow 700 Nl/h.

The chitosan microspheres obtained were soluble in an aqueous solution pH 4-5 (solution of water /acetic acid 0.5% or with 1M HCl)

### Example 5: Alginate microspheres containing lysozyme or vibrio.

12 ml of an aqueous solution of HPMC (0.4% w/v) was added to 10 g of an aqueous solution of sodium alginate (4% w/v); this was left under stirring for 5 minutes and then sonified for another 5 minutes. 0.1 g of lysozyme or *Vibrio anguillarum* previously suspended in 1 ml of water was added to the solution of alginate and HPMC and left under stirring until obtaining a homogeneous suspension. This mixture was poured dropwise into 100 ml of sunflower oil containing Arlacel 1689 (1% v/v), using a mechanical stirrer at 1000 rpm for 10 minutes. At this point 5 ml of a solution of CaCl₂ 0.5% + 1 % Tween 80 was added slowly and left under stirring for 5 minutes. Subsequently 30 ml of a solution containing anhydrous CaCl₂15% was added and left to react under stirring for 10 minutes. 60 ml of isopropanol was added to the mixture and left under stirring for 5 minutes. The emulsion obtained was centrifuged for 10 minutes at 1100 xg. It was vacuum filtered through cellulose nitrate filters with cut-off of 0.45 µm and the residue was washed with isopropanol. The filtered microcapsules were oven dried for 24h at 37°C.

The alginate microspheres were soluble in 0.5 M sodium citrate.

The content of lysozyme and antigen and the release of lysozyme at various pH as described below, was then determined on microcapsules prepared according to example 1, compared with microcapsules of example 4 and 5.

### Example 6: determination of the content of lysozyme and antigen

Samples of microcapsules containing both lysozyme and vibrio were solubilized and subsequently, after having eliminated the alginates from the solution by precipitation, the total content of lysozyme and *Vibrio anguillarum* was determined with immunoprecipitation techniques (ELISA test) and by protein quantitation using Bradford reagent; HPLC chromatography was also used only to determine the quantity of lysozyme.

### Precipitation of alginates

The samples of microspheres with a single layer of alginate were solubilized in 0.5 M sodium citrate while for those with a double layer of chitosan-alginate it was necessary first to solubilize the chitosan in acetic acid 0.5% or 1 N HCl. Subsequently, to precipitate the alginates, a volume 2/3 times greater of alcohol (isopropanol) was added. After centrifuging for 10 minutes at 550 xg at 4°C, the formation of a flocculent white pellet was observed. The supernatant alcohol solution, containing the solubilized protein, was extracted and, as the alcohol interfered in the assay, dialysis of the solution was performed. Dialysis membranes with a cut-off of 12000 Dalton were used.

Samples were dialyzed in a vessel containing distilled water, preferably under stirring, for a minimum of 24 h. Subsequently, the dialyzed product was extracted, recording the volume obtained from individual samples, and the extracted solution was concentrated until obtaining an optimal volume for the assay to be performed.

### a) ELISA Test

The total quantity of lysozyme and *Vibrio anguillarum* were determined with the ELISA test, utilizing biotinylated anti-lysozyme and anti-vibrio rabbit antibody. The following were prepared: lysozyme and vibrio solutions, dissolved in distilled water to perform the calibration curve and solutions of the samples being assayed, solubilized in the respective solvents.

A 96-well polystyrene ELISA plate was coated with 2 µg / well (for a total volume of 200 µl per well) of antigen diluted in 0.1 M bicarbonate buffer pH 9.6. The plate was incubated for one night at 4°C. The excess antigen was removed and the plate was subsequently washed three times with PBS/Tween 20 (0.1 %). After blocking of the plate with skimmed milk (2% w/v) in PBS for 1 h at 37°C and subsequent washing with PBS/Tween 20, it was incubated at 37°C for 1 hour with the biotinylated anti-lysozyme (1:1000) or anti-vibrio (1:100) antibody.

Finally, after three further washings, the plate was incubated for 30 minutes at 37°C with streptavidin combined with alkaline phosphatase, diluted 1:1000 and subsequently with the substrate for phosphatase p-nitrophenyl phosphate (PNPP) at the concentration of 1.0 mg/ml in glycine buffer pH 10.4. Readings were taken at regular intervals of time at 405 nm utilizing a BS 1000 Packard spectrophotometer.

### b) Protein quantitation using Bradford reagent

The concentration of bacterial proteins or lysozyme in the microcapsules was determined with the method described by Bradford, which allows quantitation up to 10 µg/ml. Standard solutions of BSA [1 mg/ml] utilized as positive control and solutions of lysozyme and vibrio [1 mg/ml], dissolved in water, in 0.5 M sodium citrate and in water/acetic acid, as reference for the samples assayed, were prepared to perform the calibration curves. The samples for the chitosan-lysozyme study were solubilized in water/acetic acid (0.5%), the alginate microspheres were dissolved in 0.5 M sodium citrate, while for those with a double-layer of chitosan-alginate it was necessary to first solubilize the chitosan in acetic acid 0.5% or 1N HCl. In a 96-well plate, 200 µl of Bradford mixture was added to 40 µl of the samples being assayed, suitably diluted. The spectrophotometric readings were performed at 590 nm with an ELISA plate reader (BS 1000 SpectraCount, Packard).

### c) HPLC determination

The stock solution of lysozyme was obtained by solubilizing 10 mg in 1 ml of water/acetic acid mixture (0.5%) for the chitosan-lysozyme microcapsules study; the alginate-lysozyme microcapsules were solubilized in 0.5 M sodium citrate and for those with a double layer of chitosan-alginate it was first necessary to solubilize the chitosan in acetic acid 0.5% or 1N HCl. After precipitation of the alginates using isopropanol, the alcohol solutions were dialyzed in water and subsequently assayed. These solutions were utilized every day to prepare the calibration curve at the concentration of 25, 50, 100 µg/ml. The samples being assayed, that is pure polymers and microcapsules, either empty or containing lysozyme, were dissolved in water/acetic acid 0.5%.

Once diluted in the mobile phase, 50 µl of samples were column-injected (injection loop). The concentration of lysozyme in the microcapsules was determined by UV at a wavelength of 280 nm, corresponding to maximum absorption of the analyte considered.

Chromatography assay was performed with a mobile phase constituted by a mixture of trifluoroacetic acid in acetonitrile (0.1 %) and trifluoroacetic acid in water (0.1 %) in a ratio or 1 : 3. A flow velocity of 1.2 ml/ minute was utilized.

To evaluate the validity of the method, linearity, repeatability, reproducibility and sensitivity were calculated, all of which gave excellent results.

**Table 3: fraction of effective loading, expressed in percentage, of lysozyme in the microcapsules with respect to the expected percentage.**

| Samples | Protein quantitation | ELISA test | HPLC |
|---|---|---|---|
| | using Bradford (% load) | (% load) | (% load) |
| Single-layer microcapsules | | | |
| ex. 4 chitosan-lysozyme | 94 | - | 88 |
| ex. 4 chitosan-vibrio | 88 | 84 | - |
| ex.5 alginate-lysozyme | 37 | - | 23 |
| ex. 5 alginate-vibrio | 44 | 36 | - |

| Double-layer microcapsules | | | |
|---|---|---|---|
| ex. 1 chitosan-alginate | 96 | 95 | 86 |
| lysozyme | | | |
| ex. 1 chitosan-alginate | 22 | 21 | - |
| vibrio | | | |
| ex. 1 chitosan-alginate | 30 | - | - |
| lysozyme+vibrio | | | |

### Example 7: Release of lysozyme at different pH

The microcapsules were suspended in buffers at pH 3 and 5.5 and were kept under stirring at 37°C for 24 h. At predetermined intervals of time the suspensions were then centrifuged for 5 minutes and an aliquot was taken from the supernatant to perform protein quantitation using Bradford reagent. At the end of this incubation, the 2 samples were centrifuged, the supernatant was extracted and the samples were resuspended in a buffer at pH 8 for the subsequent 24 h. In this way a long residence time in an acid environment (stomach) followed by residence in an alkaline environment (intestine) was simulated. The results obtained indicated that at pH 3 and at pH 5 the release of lysozyme after 24 h was 3.0% and 3.2% respectively (analogous calculations performed by HPLC gave 2.4% and 3.6% respectively). On the contrary, residence in an alkaline environment led to the release of around 60% of lysozyme, irrespective of whether the solution started out at pH 3 or at pH 5.5 (Table 4).

**Table 4: Percentage of lysozyme released from the microcapsules of example 1, 4, 5 after 24 hours of incubation at pH 3 and pH 5.5 and after a subsequent 24 h of incubation at pH 8 and determined by protein quantitation using Bradford reagent.**

| | pH3 | pH 5.5 | pH3-pH8 | pH5.5-pH8 |
|---|---|---|---|---|
| | *% release* | *% release* | *% release* | *% release* |
| Ex 1: chitosan-alginate lysozyme | 3.0 | 3.2 | 60.0 | 60.0 |
| Ex.4: chitosan lysozyme | 70.0 | 80.0 | - | - |
| Ex.5: alginate lysozyme | 3.0 | 4.0 | 70.0 | 70.0 |

The effects for the purposes of loading and release of lysozyme were also evaluated on microcapsules in which the components were combined together in different concentration ratios with respect to the microcapsules in Example 1 and corresponding to the data set forth in Table 2.

### Example 8: effects on the loading and release of lysozyme of the variation in the concentration of the components expressed as % w/v (chitosan, alginate, calcium chloride and HPMC)

The effective loading obtained was evaluated as follows:
1 mg of sample was solubilized in water/acetic acid (0.5%) or 1 M HCl to facilitate solubilization of chitosan and then in 0.5 M sodium citrate. The samples were then sonified repeatedly, until the suspended particles disappeared completely. After complete dissolution, a volume 2/3 times greater of isopropanol was added to precipitate the alginate. The solution was then centrifuged for 10 minutes at 550 xg at 4°C and the supernatant extracted and dialyzed in water for 24h using membranes with a cut-off of 12000 Dalton. The dialyzed samples were extracted, measuring the volume obtained, and assayed with protein quantitation using Bradford reagent and HPLC as already described previously. The results set forth in Table 5, obtained by HPLC are expressed in µg of lysozyme.

**Tab.5**

| *microcps* | *tot theoretic.*µ*g* | µ*g obtain.* | *% load* |
|---|---|---|---|
| 1 | 330 | 50.5 | 15.3 |
| 2 | 250 | 56.3 | 22.5 |
| 3 | 200 | 44.5 | 22.2 |
| 4 | 250 | 40.7 | 16.3 |
| 5 (Ex. 3) | 200 | 27.1 | 13.5 |
| 6 | 330 | 16.0 | 4.8 |
| 7 | 200 | 19.4 | 9.7 |
| 8 (Ex. 2) | 330 | 29.2 | 8.8 |
| 9 | 250 | 61.3 | 24.5 |

Lysozyme release from the microcapsules was evaluated at different pH passing from an acid environment to a basic environment to simulate the conditions found in the stomach and intestine according to the following system:
5 mg of microcapsules were suspended in 5 ml of glycine/HCl buffer pH 3; the suspension was kept under stirring at 37°C for at least 24h. An aliquot was extracted every 2h and, after centrifuging the samples for 5 minutes at 550 xg, protein quantitation using Bradford reagent was performed, on a standard curve of lysozyme in water. After around 24h the sample was centrifuged for 5 minutes at 555 xg and the supernatant extracted. The precipitated microcapsules were resuspended in 5 ml of phosphate buffer pH 8 and kept under stirring at 37°C for at least 24 h. An aliquot was taken every 2 h and, after centrifuging the samples for 5 minutes at 550 xg, protein quantitation using Bradford reagent was performed on a standard curve of lysozyme in water. To perform chromatographic assay with HPLC the solution had to be dialyzed in water to eliminate the phosphate buffer. The data relative to release after 24h at pH 3 and at pH 8 are set forth in Table 6.

**Table 6**

| | | a pH3 | | pH3 → pH8 | |
|---|---|---|---|---|---|
| *Micro capsule* | *tot µg lyso* | *µg released* | *% release* | *µg released* | *% release* |
| 1 | 252.5 | 16.0 | 6.3 | 216.7 | 85.8 |
| 2 | 281.5 | 17.9 | 6.3 | 60.6 | 21.5 |
| 3 | 222.5 | 26.9 | 12.1 | 109.1 | 49.0 |
| 4 | 203.5 | 12.0 | 5.9 | 128.3 | 63.0 |
| 5 (ex. 3) | 135.5 | 12.1 | 8.9 | 96.0 | 70.8 |
| 6 | 80.0 | 10.2 | 12.7 | 76.0 | 95.0 |
| 7 | 97.0 | 23.5 | 24.2 | 64.5 | 66.5 |
| 8 (ex. 2) | 146.0 | 20.3 | 13.9 | 32.5 | 22.3 |
| 9 | 306.5 | 22.0 | 7.2 | 73.1 | 23.8 |

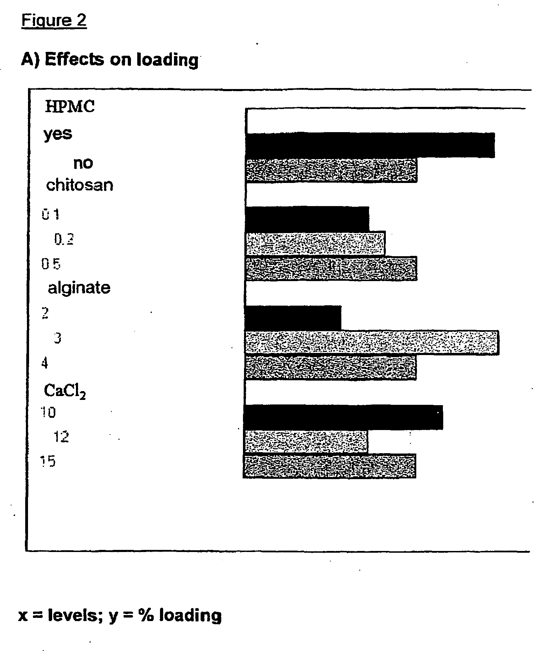

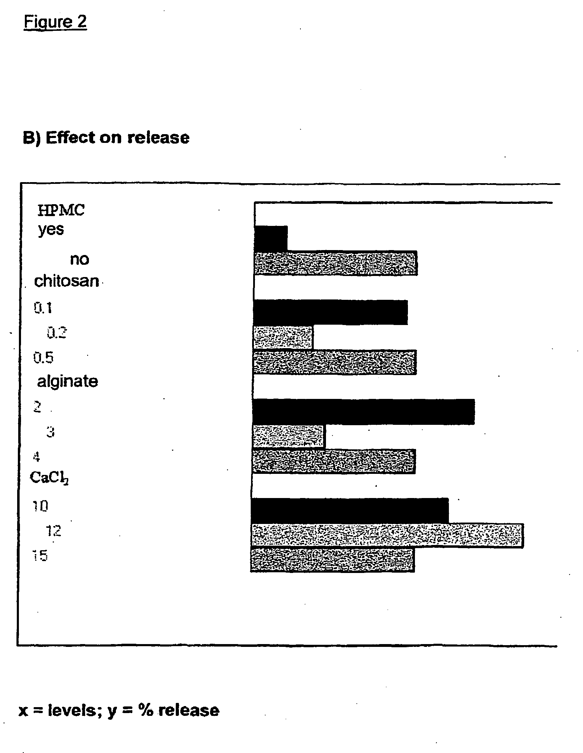

In the experimental conditions cited above the influence of HPMC was also evaluated, obtaining the results set forth in Figure 2.

By analyzing Figure 2 it is possible to establish the levels of the variables that have the greatest influence on the two characteristics of the microcapsules assayed, that is on the loading % and on the release % of the protein or antigen.

With regard to the loading % it is deduced that this is maximum in the microcapsules containing HPMC, chitosan 0.5%, alginate 3% and CaCl₂ 10%.

However, with regard to the release %, this is delayed due to the presence of the following factors: HPMC, chitosan 0.2%, alginate 3% and CaCl₂15%, while it increases in the absence of HPMC, with chitosan 0.5%, alginate 2% and CaCl₂ 12%.

For microcapsules obtained in example 1 encapsulating lysozyme and vibrio or only lysozyme and only vibrio some characteristics (diameter, solubility and degree of swelling) were compared with microcapsules containing only chitosan or only alginate prepared according to examples 4 and 5.

### Example 9: Characterization of the microcapsules of example 1, 4, 5

### Mean diameter

Determination of the particle distribution and of the mean diameter of the microcapsules was performed using image analysis technique, utilizing an optical microscope (Olympus BH-2) connected to a computerized system (Optomax-W, Cambridge).

The chitosan microcapsules, containing lysozyme, had a mean diameter of 3 µm, also found for those with *Vibrio anguillarum,* while the alginate microspheres had a mean diameter of 7 µm, relative to those containing lysozyme and of 8 µm in those with vibrio. The microcapsules constituted by a double layer of chitosan and alginate maintained the dimensions required for uptake at intestinal level (diameter < 10 µm) (Table 7).

### Degree of swelling

Again using the image analysis technique, the tendency to swell of the microcapsules, placed in water for 12h, was evaluated, due partly to uptake of the liquid by the polymer and by HPMC contained in the preparation. Subsequently, distribution of the particle diameter of the "swollen" microspheres was measured and the increase observed for the various microspheres was of around 2 µm, a value that in any case comes within the required limits.

### Solubility

As indicated in Table 7 the chitosan microcapsules were soluble in an acid solution at pH 4-5 of water/acetic acid (0.5%) or in 1M hydrochloric acid, while the alginate microcapsules were solubilized in 0.5M sodium citrate. The double-layer microcapsules were solubilized in water/acetic acid (0.5%) or 1M HCl to facilitate solubilization of the chitosan and then in 0.5M sodium citrate.

**Table 7**

| Samples I | Mean diameter (µm) | Solubility |
|---|---|---|
| Single-layer microcapsules | | |
| ex. 4 chitosan-lysozyme | 3 | water/acetic acid or 1M HCl |
| ex. 4 chitosan-vibrio | 3 | water/acetic acid or 1M HCl |
| ex.5 alginate-lysozyme | 7 0.5M | sodium citrate |
| ex. 5 alginate-vibrio | 8 0.5M | sodium citrate |

| Double-layer microcapsules | | |
|---|---|---|
| ex. 1 chitosan-alginate lysozyme | 5 | water/acetic acid or 1M HCl -0.5M sodium citrate |
| ex. 1 chitosan-alginate vibrio | 5 | water/acetic acid or 1M HCl - 0.5M sodium citrate |
| ex. 1 chitosan-alginate lysozyme+vibrio | 5 | water/acetic acid or 1M HCl - 0.5M sodium citrate |

### Example 10: Activation of the immune system in vivo

Evaluation of the capacity of the double-layer microcapsule of example 1 to deliver the antigen and the adjuvant in the immune system of the animal after oral administration was performed on a murine model utilizing female CBA mice, to which the microcapsules were administered through food. In brief, double-layer microcapsules containing lysozyme and/or vibrio, corresponding in weight to the administration of 2 mg/kg/day of lysozyme and 1 mg (1.5x10⁹ cells) of vibrio/mouse/day, were mixed with powdered food and administered to groups of animals previously conditioned to being fed the food in question. For the control, groups were utilized treated with free lysozyme or Vibrio or with microcapsules containing only lysozyme or only vibrio respectively. The immunization treatment was protracted for 6 consecutive days in groups of 5 animals.

**Table 8**

| Groups | | lgM after immunization | |
|---|---|---|---|
| | | 24h | 7gg |
| Anti-lysozyme antibodies | | | |
| controls | | 432±69 | 478±49 |
| lysozyme | | 743±69* | 836±141 |
| chito/alg-lysozyme | | 824±25* | 592±150 |
| chito/alg-lyso/vibrio | | 1291±104** | 616±56 |

| Anti-vibrio antibodies | | | |
|---|---|---|---|
| controls | | 548±126 | 508±55 |
| vibrio | | 762±142 | 512±128 |
| chito/alg-vibrio | | 1192±138* | 619±100 |
| chito/alg-lysozyme/vibrio | | 1417±192* | 890±74* |

Table 8. Antibodies measured by ELISA test in the sera of the animals tested after taking small blood samples from the cheek pouch. Each value indicated is the average ± standard error of 5 different animals.

The data set forth in Table 8 clearly show that the double-layer microcapsule, loaded with adjuvant (lysozyme) and antigen (vibrio) is able to induce much greater stimulation of the immune responses, measured by the quantity of IgM released, than obtained when a single component is loaded, both as regards anti-lysozyme antibodies and when compared to anti-vibrio antibodies. The effect is decidedly more marked if compared with administration of non-microencapsulated lysozyme and vibrio (*p<0.05, **p<0.01, analysis of variance: Anova and Student Newmann Keuls post test).

The microcapsules of the invention, having a diameter below 10 µm, formed by an inner layer of alginate containing the corpuscular antigen to be delivered to the immune system by means of oral administration and an outer layer of chitosan which guarantees properties of mucoadhesion to the intestinal wall, are efficacious to distribute to the mucosal immune system the antigen and the adjuvant, represented in the case illustrated by lysozyme (Table 8).

The study shows that double-layer microcapsules, as proposed herein, are also able to protect the antigen from degrading effects in the stomach of the animals treated, proving resistant to degradation at acid pH and facilitating, on the contrary, release of its content at alkaline pH, as is found in the first part of the intestine which, both in the case of mammals and in fish, is full in lymphoid organs. The microcapsules of the invention can thus be usefully employed as carriers of biologically active substances for vaccinogenic purposes for the prophylaxis and therapy of infectious and non-infectious pathologies or for therapeutic purposes of these in the human and veterinary field.

The microcapsules of the invention can be administered in compositions with suitable excipients or diluents acceptable in the pharmaceutical or food field and for the use established and in forms suitable for the purpose such as solid forms (powders, tablets, capsules) or in liquid forms (oily or aqueous solutions) both for multiple dosage and in single doses.

In particular, in the veterinary field, in animal breeding or in fish farming, the microcapsules of the invention can also be administered in the form of powders mixed with the foods employed to feed the animals.

## Claims

1. Polysaccharide double-layer microcapsules constituted by an outer layer of chitosan and an inner layer of alginate **characterized in that** they are obtained:
from solutions of alginate with initial concentrations ranging from 2 to 4% w/v;
- from solutions of chitosan with initial concentrations ranging from 0.1 to 0.5 % w/v;
- from solutions of divalent ions with concentrations of 0.5% w/v, when the divalent ion functions as a gelification agent of the alginate to form single-layer capsules of alginate encapsulating at least one biologically active substance, and ranging from 10 to 15% w/v when the divalent ion has a stabilizing function of the double layer capsules for use as carriers for the oral administration of said biologically active substances.

2. Polysaccharide double-layer microcapsules as claimed in claim 1, wherein a further polymer, hydroxypropylmethylcellulose, at the initial concentration of 0.4% w/v, is dispersed in the initial solutions of alginate.

3. Polysaccharide double-layer microcapsules as claimed in claim 1, wherein the initial concentration of alginate is 4% w/v, the initial concentration of chitosan is 0.1% w/v and the divalent ion with stabilizing function on the double layer microcapsules has an initial concentration of 15% w/v.

4. Polysaccharide double-layer microcapsules as claimed in claim 1, wherein the divalent ion is calcium.

5. Polysaccharide double-layer microcapsules as claimed in claim 1, wherein the biologically active substances are selected from immunomodulants, antigens, chemotherapeutics, cytokines and growth factors.

6. Polysaccharide double-layer microcapsules as claimed in claim 1, wherein an adjuvant is associated with the biologically active substance to increase the biological response.

7. Polysaccharide double-layer microcapsules as claimed in claim 1 and 6, wherein the biologically active substance or adjuvant is lysozyme.

8. Polysaccharide double-layer microcapsules constituted by an outer layer of chitosan and an inner layer of alginate **characterized in that** they are obtained through:
a) formation of single-layer capsules encapsulating at least one biologically active substance starting from solutions of alginate in concentrations ranging from 2 to 4% w/v, in which said substance is dispersed, by gelification with a solution of a divalent ion at a concentration of 0.5% w/v;
b) formation of the second layer of chitosan and stabilization of the double-layer microcapsule obtained by adding a solution of chitosan in concentrations ranging from 0.1 to 0.5% w/v and containing a divalent ion in concentrations ranging from 10 to 15% w/v in the solution containing the single-layer capsules of alginate encapsulating said substance obtained in a).
for use as carriers for the oral administration of said biologically active substances.

9. Polysaccharide double-layer microcapsules as claimed in claim 8, wherein a further polymer, hydroxypropylmethylcellulose, at the initial concentration of 0.4% w/v, is dispersed in the initial solutions of alginate.

10. Polysaccharide double-layer microcapsules as claimed in claim 8, wherein the initial concentration of alginate is 4% w/v, the initial concentration of chitosan is 0.1% w/v and the divalent ion with stabilizing function on the double layer microcapsules has an initial concentration of 15% w/v.

11. Polysaccharide double-layer microcapsules as claimed in claim 8, wherein the divalent ion is calcium.

12. Polysaccharide double-layer microcapsules as claimed in claim 8, wherein the biologically active substances are chosen from immunomodulants, antigens, chemotherapeutics, cytokines and growth factors.

13. Polysaccharide double-layer microcapsules as claimed in claim 8, wherein an adjuvant is associated with the biologically active substance to increase the biological response.

14. Polysaccharide double-layer microcapsules as claimed in claim 8 and 13, wherein the biologically active substance or adjuvant is lysozyme.

15. Use of polysaccharide double-layer microcapsules as claimed in one of the claims from 1 to 14 for the preparation of compositions for oral administration of at least one biologically active substance for vaccinogenic or therapeutic purposes for the prophylaxis and therapy of infectious or non-infectious diseases in the human and veterinary field.

16. Use of polysaccharide double-layer microcapsules as claimed in claim 15 for the prophylaxis and therapy in the animal breeding or fish farming field.

17. Composition of polysaccharide double-layer microcapsules as claimed in one of the claims from 1 to 14, in formulations suitable for oral administration selected from solid forms consisting of powders, tablets, capsules, or liquid forms consisting of oily or aqueous solutions, both for multiple dosage and in single doses with excipients or diluents acceptable from the pharmaceutical and feeding purposes in the human and veterinary field.

18. Process for preparation of polysaccharide double-layer microcapsules constituted by an outer layer of chitosan and an inner layer of alginate **characterized in that** it comprises the following phases:
a) formation of single-layer capsules encapsulating at least one biologically active substance starting from solutions of alginate in concentrations ranging from 2 to 4% w/v, in which said substance is dispersed, by gelification with a solution of a divalent ion at a concentration of 0.5% w/v;
b) formation of the second layer of chitosan and stabilization of the double-layer microcapsule obtained by adding a solution of chitosan in concentrations ranging from 0.1 to 0.5% w/v and containing a divalent ion in concentrations ranging from 10 to 15% w/v in the solution containing the single-layer capsules of alginate encapsulating at least one biologically active substance obtained in a).

19. Process for preparation of polysaccharide double-layer microcapsules as claimed in claim 18, wherein added to phases a) and b) is the phase c) of dehydration, isolation and drying of the microcapsules obtained.

20. Process for preparation of polysaccharide double-layer microcapsules as claimed in claim 18, wherein a further polymer, hydroxypropylmethylcellulose, at the initial concentration of 0.4% w/v, is dispersed in the initial solutions of alginate.

21. Process for preparation of polysaccharide double-layer microcapsules as claimed in claim 18, wherein the initial concentration of alginate is 4% w/v, the initial concentration of chitosan is 0.1% w/v and the divalent ion with stabilizing function on the double layer microcapsules has an initial concentration of 15% w/v.

22. Process for preparation of polysaccharide double-layer microcapsules as claimed in claim 18, wherein the divalent ion is calcium.

23. Process for the preparation of microcapsules as claimed in claim 18, wherein the biologically active substances are chosen from immunomodulants, antigens, chemotherapeutics, cytokines and growth factors.

24. Process for preparation of polysaccharide double-layer microcapsules as claimed in claim 18, wherein an adjuvant is associated with the biologically active substance to increase the biological response.

25. Process for preparation of polysaccharide double-layer microcapsules as claimed in claims 18 and 24, wherein the biologically active substance or adjuvant is lysozyme.

## Patentansprüche

1. Polysaccharid-Doppelschicht-Mikrokapseln, welche aus einer äußeren Schicht aus Chitosan und einer inneren Schicht aus Alginat bestehen, und **dadurch gekennzeichnet sind, dass** sie gewonnen werden aus:
Lösungen von Alginat mit Anfangskonzentrationen im Bereich von 2 bis 4 Gramm-Vol.%;
Lösungen von Chitosan mit Anfangskonzentrationen im Bereich von 0,1 bis 0,5 Gramm-Vol.%;
Lösungen von bivalenten Ionen mit Konzentrationen von 0,5 Gramm-Vol.%, wenn das bivalente Ion als eine gelierende Substanz von Alginat wirkt, um Einschicht-Kapseln aus Alginat zu bilden, welche mindestens eine biologische Wirksubstanz enthalten, und mit Konzentrationen im Bereich von 10 bis 15 Gramm-Vol. %, wenn das bivalente Ion eine stabilisierende Wirkung auf die Doppelschicht-Kapseln ausübt,
für die Verwendung als Trägerstoffe zur oralen Verabreichung von genannten biologischen Wirksubstanzen.

2. Polysaccharid-Doppelschicht-Mikrokapseln gemäß Anspruch 1, wobei ein weiteres Polymer, Hydroxypropylmethylcellulose, mit einer Anfangskonzentration von 0,4 Gramm-Vol.% in den Anfangslösungen von Alginat dispergiert ist.

3. Polysaccharid-Doppelschicht-Mikrokapseln gemäß Anspruch 1, wobei die Anfangskonzentration von Alginat 4 Gramm-Vol.%, die Anfangskonzentration von Chitosan 0,1 Gramm-Vol.% und das bivalente Ion mit stabilisierender Wirkung auf die Doppelschicht-Mikrokapseln eine Anfangskonzentration von 15 Gramm-Vol.% aufweist.

4. Polysaccharid-Doppelschicht-Mikrokapseln gemäß Anspruch 1, wobei es sich bei dem bivalenten Ion um Kalzium handelt.

5. Polysaccharid-Doppelschicht-Mikrokapseln gemäß Anspruch 1, wobei die biologischen Wirksubstanzen ausgewählt sind unter immunmodulierenden Substanzen, Antigenen, Chemotherapeutika, Zytokinen und Wachstumsfaktoren.

6. Polysaccharid-Doppelschicht-Mikrokapseln gemäß Anspruch 1, wobei ein Adjuvans mit der biologischen Wirksubstanz assoziiert ist, um die biologische Reaktion zu verstärken.

7. Polysaccharid-Doppelschicht-Mikrokapseln gemäß Anspruch 1 und Anspruch 6, wobei es sich bei der biologischen Wirksubstanz oder dem Adjuvans um Lysozym handelt.

8. Polysaccharid-Doppelschicht-Mikrokapseln, welche aus einer äußeren Schicht aus Chitosan und einer inneren Schicht aus Alginat bestehen, und **dadurch gekennzeichnet sind, dass** sie gewonnen werden durch:
a) Bilden von Einschicht-Kapseln, welche mindestens eine biologische Wirksubstanz enthalten, und welche von Lösungen von Alginat mit Konzentrationen im Bereich von 2 bis 4 Gramm-Vol.% ausgehen, in welchen die genannte Substanz dispergiert ist, durch Gelieren mit einer Lösung eines bivalenten Ions mit einer Konzentration von 0,5 Gramm-Vol.%;
b) Bilden der zweiten Schicht aus Chitosan und Stabilisieren der erhaltenen Doppelschicht-Mikrokapsel, indem eine Lösung von Chitosan in Konzentrationen im Bereich von 0,1 bis 0,5 Gramm-Vol.% zugegeben wird, und welche ein bivalentes Ion in Konzentrationen im Bereich von 10 bis 15 Gramm-Vol.% in der Lösung enthält, welche die Einschicht-Kapseln aus Alginat, die in a) erhalten wurden, welche die genannte Substanz beinhalten, umfasst,
für die Verwendung als Trägerstoffe zur oralen Verabreichung der genannten biologischen Wirksubstanzen.

9. Polysaccharid-Doppelschicht-Mikrokapseln gemäß Anspruch 8, wobei ein weiteres Polymer, Hydroxypropylmethylcellulose, mit einer Anfangskonzentration von 0,4 Gramm-Vol.% in den Anfangslösungen von Alginat dispergiert ist.

10. Polysaccharid-Doppelschicht-Mikrokapseln gemäß Anspruch 8, wobei die Anfangskonzentration von Alginat 4 Gramm-Vol.% beträgt und die Anfangskonzentration von Chitosan bei 0,1 Gramm-Vol.% liegt und das bivalente Ion mit stabilisierender Wirkung auf die Doppelschicht-Mikrokapseln eine Anfangskonzentration von 15 Gramm-Vol.% aufweist.

11. Polysaccharid-Doppelschicht-Mikrokapseln gemäß Anspruch 8, wobei es sich bei dem bivalenten Ion um Kalzium handelt.

12. Polysaccharid-Doppelschicht-Mikrokapseln gemäß Anspruch 8, wobei die biologischen Wirksubstanzen ausgewählt sind unter immunmodulierenden Substanzen, Antigenen, Chemotherapeutika, Zytokinen und Wachstumsfaktoren.

13. Polysaccharid-Doppelschicht-Mikrokapseln gemäß Anspruch 8, wobei ein Adjuvans mit der biologischen Wirksubstanz assoziiert ist, um die biologische Reaktion zu verstärken.

14. Polysaccharid-Doppelschicht-Mikrokapseln gemäß Anspruch 8 und Anspruch 13, wobei es sich bei der biologischen Wirksubstanz oder dem Adjuvans um Lysozym handelt.

15. Verwendung von Polysaccharid-Doppelschicht-Mikrokapseln gemäß einem der Ansprüche von 1 bis 14 für die Herstellung von Zusammensetzungen für die orale Verabreichung von mindestens einer biologischen Wirksubstanz zu Impfzwecken oder therapeutischen Zwecken für die Prophylaxe und Therapie von infektiösen oder nicht infektiösen Erkrankungen auf dem humanmedizinischen und veterinärmedizinischen Gebiet.

16. Verwendung von Polysaccharid-Doppelschicht-Mikrokapseln gemäß Anspruch 15 für die Prophylaxe und Therapie auf dem Gebiet der Tierzucht und der Fischzucht.

17. Zusammensetzung von Polysaccharid-Doppelschicht-Mikrokapseln gemäß einem der Ansprüche von 1 bis 14 in Formulierungen, welche für die orale Verabreichung geeignet und ausgewählt sind unter Festformen, welche aus Pulvern, Tabletten und Kapseln bestehen, oder unter Flüssigformen, welche aus öligen oder wässrigen Lösungen bestehen, sowohl für Mehrfachdosierungen als auch Einzeldosierungen mit Hilfsstoffen oder Verdünnungsmitteln, welche für den pharmazeutischen Verwendungszweck und die Verwendung bei der Fütterung anerkannt sind.

18. Verfahren für die Herstellung von Polysaccharid-Doppelschicht-Mikrokapseln, welche durch eine äußere Schicht aus Chitosan und eine innere Schicht aus Alginat gebildet werden, und wobei das Verfahren **dadurch gekennzeichnet ist, dass** es die folgenden Abschnitte umfasst:
a) Bilden von Einzelschicht-Kapseln, welche mindestens eine biologische Wirksubstanz enthalten, welche von Lösungen von Alginat mit Konzentrationen im Bereich von 2 bis 4 Gramm-Vol.% ausgehen, in welchen die genannte Substanz dispergiert ist, durch Gelieren mit einer Lösung eines bivalenten Ions mit einer Konzentration von 0,5 Gramm-Vol.%.
b) Bilden der zweiten Schicht aus Chitosan und Stabilisieren der erhaltenen Doppelschicht-Mikrokapsel, indem eine Lösung von Chitosan in Konzentrationen im Bereich von 0,1 bis 0,5 Gramm-Vol.% zugegeben wird, und welche ein bivalentes Ion in Konzentrationen im Bereich von 10 bis 15 Gramm-Vol.% in der Lösung enthält, welche die Einschicht-Kapseln aus Alginat, die in a) erhalten wurden, welche mindestens eine biologische Wirksubstanz, beinhalten, umfasst.

19. Verfahren für die Herstellung von Polysaccharid-Doppelschicht-Mikrokapseln gemäß Anspruch 18, wobei zu Abschnitt a) und Abschnitt b) der Abschnitt c) mit Wasserentzug, Isolierung und Trocknung der erhaltenen Mikrokapseln hinzukommt.

20. Verfahren für die Herstellung von Polysaccharid-Doppelschicht-Mikrokapseln gemäß Anspruch 18, wobei ein weiteres Polymer, Hydroxypropylmethylcellulose, mit einer Anfangskonzentration von 0,4 Gramm-Vol.% in den Anfangslösungen von Alginat dispergiert ist.

21. Verfahren für die Herstellung von Polysaccharid-Doppelschicht-Mikrokapseln gemäß Anspruch 18, wobei die Anfangskonzentration von Alginat, bei 4 Gramm-Vol.% liegt, die Anfangskonzentration von Chitosan 0,1 Gramm-Vol.% beträgt und das bivalente Ion mit stabilisierender Wirkung auf die Doppelschicht-Mikrokapseln eine Anfangskonzentration von 15 Gramm-Vol.% aufweist.

22. Verfahren für die Herstellung von Polysaccharid-Doppelschicht-Mikrokapseln gemäß Anspruch 18, wobei es sich bei dem bivalenten Ion um Kalzium handelt.

23. Verfahren für die Herstellung von Polysaccharid-Doppelschicht-Mikrokapseln gemäß Anspruch 18, wobei die biologischen Wirksubstanzen ausgewählt sind unter immunmodulierenden Substanzen, Antigenen, Chemotherapeutika, Zytokinen und Wachstumsfaktoren.

24. Verfahren für die Herstellung von Polysaccharid-Doppelschicht-Mikrokapseln gemäß Anspruch 18, wobei ein Adjuvans mit der biologischen Wirksubstanz assoziiert ist, um die biologische Reaktion zu verstärken.

25. Verfahren für die Herstellung von Polysaccharid-Doppelschicht-Mikrokapseln gemäß Anspruch 18 und Anspruch 24, wobei es sich bei der biologischen Wirksubstanz oder dem Adjuvans um Lysozym handelt.

## Revendications

1. Microcapsules à double couche en polysaccharide constituées d'une couche externe de chitosan et d'une couche interne d'un alginate **caractérisées en ce qu'**elles sont obtenues :
à partir de solutions d'alginate avec des concentrations initiales comprises entre 2 et 4% p/v ;
à partir de solutions de chitosan avec des concentrations initiales comprises entre 0,1 et 0,5% pv ;
à partir de solutions d'ions divalents à des concentrations de 0,5% p/v, quand l'ion divalent fonctionne comme un agent de gélification de l'alginate pour former des capsules monocouche d'alginate en encapsulant au moins une substance biologiquement active et comprise entre 10 et 15% p/v quand l'ion divalent a une fonction de stabilisation des capsules double couche à utiliser comme support pour administration orale desdites substances biologiquement actives.

2. Microcapsules à double couche en polysaccharide selon la revendication 1, où un autre polymère, de l'hydroxypropylméthylcellulose, à la concentration initiale de 0,4% p/v, est dispersé dans les solutions initiales de l'alginate.

3. Microcapsules à double couche en polysaccharide selon la revendication 1, où la concentration initiale de l'alginate est de 4% p/v, la concentration initiale du chitosan est de 0,1% p/v et l'ion divalent ayant une fonction de stabilisation sur les microcapsules à double couche a une concentration initiale de 15% p/v.

4. Microcapsules à double couche en polysaccharide selon la revendication 1, où l'ion divalent est du calcium.

5. Microcapsules en polysaccharide double couche selon la revendication 1, où les substances biologiquement actives sont sélectionnées parmi des immunomodulants, des antigènes, des agents chimiothérapeutiques, des cytokines et des facteurs de croissance.

6. Microcapsules à double couche en polysaccharide selon la revendication 1, où un adjuvant est associé à la substance biologiquement active pour augmenter la réponse biologique.

7. Microcapsules à double couche en polysaccharide selon la revendication 1 et 6, où la substance biologiquement active ou adjuvant est un lysozyme.

8. Microcapsules à double couche en polysaccharide constituées d'une couche externe de chitosan et d'une couche interne d'alginate **caractérisées en ce qu'**elles sont obtenues par :
a) formation de capsules en une couche encapsulant au moins une substance biologiquement active en partant de solutions d'alginate à des concentrations comprises entre 2 et 4% p/v, dans lesquelles ladite substance est dispersée, par gélification avec une solution d'un ion divalent à une concentration de 0,5% p/v ;
b) formation de la seconde couche de chitosan et stabilisation de la microcapsule à double couche obtenue par addition d'une solution de chitosan à des concentrations comprises entre 0,1 et 0,5% p/v et contenant un ion divalent à des concentrations comprises entre 10 et 15% p/v dans la solution contenant les capsules de l'alginate encapsulant ladite substance obtenues en a).
pour une utilisation comme supports pour l'administration orale desdites substances biologiquement actives.

9. Microcapsules à double couche en polysaccharide selon la revendication 8, où un autre polymère, de l'hydroxypropylméthylcellulose, à la concentration initiale 0,4% p/v, est dispersé dans les solutions initiales de l'alginate.

10. Microcapsules à double couche en polysaccharide selon la revendication 8, où la concentration initiale de l'alginate est de 4% p/v, la concentration initiale du chitosan est de 0,1% p/v et l'ion divalent avec une fonction stabilisante sur les microcapsules à double couche à une concentration initiale de 15% p/v.

11. Microcapsules à double couche en polysaccharide selon la revendication 8, où l'ion divalent est du calcium.

12. Microcapsules à double couche en polysaccharide selon la revendication 8, où les substances biologiquement actives sont choisies parmi des immunomodulants, des antigènes, des agents chimiothérapeutiques, des cytokines et des facteurs de croissance.

13. Microcapsules à double couche en polysaccharide selon la revendication 8, où un adjuvant est asocié à la substance biologiquement active pour augmenter la réponse biologique.

14. Microcapsules à double couche en polysaccharide selon la revendication 8 et 13, où la substance biologiquement active ou adjuvant est un lysozyme.

15. Utilisation de microcapsules à double couche en polysaccharide selon l'une des revendications 1 à 14, pour la préparation de compositions pour administration orale d'au moins une substance biologiquement active dans des buts vaccinogènes ou thérapeutiques pour la prophylaxie et la thérapie de maladies infectieuses ou non infectieuses dans le domaine humain et vétérinaire.

16. Utilisation de microcapsules à couche double en polysaccharide selon la revendication 15 pour la prophylaxie et la thérapie dans le domaine de l'élevage des animaux ou de l'élevage des poissons.

17. Composition de microcapsules à double couche en polysaccharide selon l'une quelconque des revendications 1 à 14, dans des formulations appropriées pour une administration orale, sélectionnées parmi des formules solides consistant en poudres, comprimés, capsules ou des formes liquides consistant en solutions huileuses et aqueuses, pour un dosage multiple en ou dose individuelle avec des excipients ou diluants acceptables dans des buts pharmaceutiques et d'alimentation dans le domaine humain et vétérinaire.

18. Procédé pour la préparation de microcapsules à double couche en polysaccharide constituées d'une couche externe de chitosan et d'une couche interne d'un alginate **caractérisé en ce qu'**il comprend les phases suivantes :
a) formation de capsules en une couche encapsulant au moins une substance biologiquement active en partant de solutions d'alginate à des concentrations comprises entre 2 et 4% p/v dans lesquelles ladite substance est dispersée par gélification avec une solution d'un ion divalent à une concentration de 0,5% p/v ;
b) formation de la seconde couche de chitosan et stabilisation de la microcapsule à double couche obtenue par addition d'une solution de chitosan à des concentrations comprises entre 0,1 et 0,5% p/v et contenant un ion divalent à des concentrations comprises entre 10 et 15% p/v dans la solution contenant les capsules à une seule couche d'alginate encapsulant une substance biologiquement active obtenues en a).

19. Procédé pour la préparation de microcapsules à double couche en polysaccharide selon la revendication 18, où est ajoutée aux phases a) et b), la phase c) de déshydratation, isolement et séchage des microcapsules obtenues.

20. Procédé de préparation de microcapsules à double couches en polysaccharide selon la revendication 8 où un autre polymère, de l'hydroxypropylméthylcellulose, à la concentration initiale de 0,4% p/v, est dispersé dans les solutions initiales d'alginate.

21. Procédé pour la préparation de microcapsules à double couche en polysaccharide selon la revendication 18, où la concentration initiale de l'alginate est de 4% p/v, la concentration initiale du chitosan est de 0,1% p/v et l'ion divalent avec une fonction de stabilisation sur les microcapsules à double couche a une concentration initiale de 15% p/v.

22. Procédé pour la préparation de microcapsules à double couche en polysaccharide selon la revendication 18, où l'ion divalent est du calcium.

23. Procédé pour la préparation de microcapsules selon la revendication 18, où les substances biologiquement actives sont choisies parmi des immunomodulants, des antigènes, des agents chimiothérapeutiques, des cytokines et des facteurs de croissance.

24. Procédé pour la préparation de microcapsules à double couche en polysaccharide selon la revendication 18, où un adjuvant est associé à la substance biologiquement active pour augmenter la réponse biologique.

25. Procédé pour la préparation de microcapsules à double couche en polysaccharide selon les revendications 18 et 24, où la substance biologiquement active ou adjuvant est un lysozyme.
